# EUROPEAN PATENT SPECIFICATION

(11) **EP 2 401 004 B1**
(45) Date of publication and mention of the grant of the patent: **18.07.2018**
(21) Application number: 10746790.4
(22) Date of filing: 24.02.2010
(51) Int. Cl.: A61N 1/39, A61N 1/362

(54) **SYSTEM FOR TREATING CARDIAC ARRHYTHMIAS**
SYSTEM ZUR BEHANDLUNG VON HERZRHYTHMUSSTÖRUNGEN
SYSTÈME DE TRAITEMENT DES ARYTHMIES CARDIAQUES

(30) Priority: 25.02.2009 US 393014; 19.02.2010 US 709433
(43) Date of publication of application: 04.01.2012
(73) Proprietor: University of Utah Research Foundation, Salt Lake City, UT 84108 (US)
(72) Inventor: HAMDAN, Mohamed, Hussein, Salt Lake City UT 84108 (US)
(74) Representative: Dunlop, Hugh Christopher
(86) International application number: PCT/US2010/025276
(87) International publication number: WO 2010/099233

(56) References cited:
- US-A- 4 830 006
- US-A1- 5 184 615
- US-A1- 2004 215 258
- US-A1- 2005 149 125
- US-A1- 2006 259 082
- US-A1- 2008 147 138
- US-A1- 2008 183 228
- US-B2- 7 206 633
- JOHN F. MACGREGOR ET AL: "The Magnitude of Sinus Cycle Length Change During Ventricular Tachycardia is Predictive of Successful Antitachycardia Pacing", PACING AND CLINICAL ELECTROPHYSIOLOGY, vol. 29, no. 11, November 2006 (2006-11), pages 1195-1200, XP055088638, ISSN: 0147-8389, DOI: 10.1111/j.1540-8159.2006.00522.x
- STEPHEN L. WASMUND ET AL: "Modulation of the Sinus Rate during Ventricular Fibrillation", JOURNAL OF CARDIOVASCULAR ELECTROPHYSIOLOGY, vol. 20, no. 2, February 2009 (2009-02), pages 187-192, XP055088681, ISSN: 1045-3873, DOI: 10.1111/j.1540-8167.2008.01314.x

## Description

### Field of the Inventions

The disclosure relates generally to systems for cardiac complications, and in particular to systems for treating cardiac arrhythmias.

### Background of the Inventions

Cardiac defibrillators are medical devices for treating patients who have experienced an episode of ventricular tachycardia or ventricular fibrillation. Cardiac defibrillators are often implanted within a patient to detect and treat ventricular tachycardia or ventricular fibrillation. Implantable cardioverter-defibrillators (ICDs) include a small battery powered electrical impulse generator that is implanted in patients who are at risk of developing ventricular fibrillation. The ICDs are programmed to detect cardiac arrhythmia and correct it by delivering a jolt of electricity through electrodes that are introduced into the heart.

ICDs can keep a record of the heart's activity when an abnormal heart rhythm occurs. With this information, an electrophysiologist can study the heart's activity and ask about other symptoms that may have occurred. Sometimes the ICD can be programmed to pace the heart to restore its natural rhythm.

US2008183228 describes an implantable cardioverter/defibrillator (ICD) that delivers atrial pacing under several scenarios during a tachyarrhythmia episode that is detected using a ventricular rate.

US2004215258 describes systems and methods that provide for sensing of cardiac activity from a subcutaneous, non-intrathoracic location, and detecting of a cardiac condition necessitating treatment in response to the sensed cardiac activity.

US2005149125 describes a cardiac rhythm management device configured to discriminate between ventricular and supraventricular tachycardias by utilizing a morphology criterion in which the morphology of electrogram waveforms during ventricular beats are analyzed to determine if the beats are normally conducted.

US4830006 describes an implantable cardiac stimulator that integrates the functions of bradycardia and anti-tachycardia pacing-type therapies, and cardioversion and defibrillation shock-type therapies.

"The Magnitude of Sinus Cycle Length Change During Ventricular Tachycardia is Predictive of Successful Antitachycardia Pacing", John F. Macgregor et al, Pacing and Clinical Electrophysiology, vol. 29, no. 11, November 2006, pages 1195-1200, XP055088638, ISSN: 0147-8389, DOI: 10.1111/j. 1540-8159.2006.00522.x is a paper investigating the hypothesis that the degree of sympathoexcitation, as measured by the sinus cycle length (SCL) shortening during ventricular tachycardia (VT), is a predicator of ATP success.

### Summary of the Inventions

In accordance with the present invention, an implantable cardiac device is provided in accordance with claim 1. Preferred embodiments are defined by the dependent claims.

Aspects, embodiments or examples of the present disclosure which do no fall within the scope of the appended claims do not form part of the present invention. Some examples described herein relate to a method of treating a fast ventricular arrhythmia that includes determining a value of a parameter indicative of a rate of an intrinsic pacemaker of a heart of a patient experiencing a fast ventricular arrhythmia (FYA); if the value indicates the rate is about equal to or higher than a threshold, delivering a first therapy to the patient for terminating the FVA; and if the value indicates the rate is lower than the threshold, delivering a second therapy, different from the first therapy, to the patient for terminating the FVA.

In some embodiments, the rate is a depolarization rate. In some embodiments, the intrinsic pacemaker comprises the sinoatrial node of the patient. In certain embodiments, the parameter indicative of the rate comprises a sinus node cycle length. Some embodiments provide that the fast ventricular arrhythmia comprises ventricular tachycardia, and the ventricular tachycardia can has a cycle length equal to or less than about 240 ms.

In some embodiments, determining a value of a parameter indicative of a rate comprises analyzing an atrial electrogram of the patient. Some embodiments provide that determining a value of a parameter indicative of a rate is performed using an electrode positioned in the patient's heart. In certain embodiments, determining a value of a parameter indicative of a rate is performed using an electrode positioned in an atrium of the patient.

Some embodiments provide that the first therapy comprises a therapy that is relatively painless to the patient. In some embodiments, the first therapy comprises anti-tachycardia pacing. In some embodiments, the second therapy comprises at least one of defibrillation and electrical cardioversion. Some embodiments provide that the second therapy comprises defibrillation. In some embodiments, the first therapy comprises anti-tachycardia pacing, and the second therapy comprises at least one of defibrillation and electrical cardioversion.

In some embodiments, determining a value of a parameter indicative of a rate and the delivering steps, mentioned above, are performed by a device implanted in the patient. In some embodiments, wherein the parameter indicative of a rate comprises a sinus node cycle length. The first therapy can include anti-tachycardia pacing, and the second therapy can include at least one of defibrillation and electrical cardioversion. The determining a value of a parameter indicative of a rate and the delivering steps can be, with the above description, performed by a device implanted in the patient.

In some embodiments, if the value indicates the rate is lower than a threshold, some methods provide for delivering a third therapy that stimulates the patient's sympathetic nervous system. In certain embodiments, the third therapy is sufficient to raise an arterial blood pressure in the patient. Some embodiments provide that the third therapy is electrical. In some embodiments, the third therapy comprises delivery of a sympathetic or sympathomimetic agent to the patient.

Some examples provide a method, of treating a fast ventricular arrhythmia, including determining a value of a parameter indicative of at least one of vagal activity and peripheral sympathetic activity in a patient experiencing a fast ventricular arrhythmia; if the value is in a range indicating the ventricular arrhythmia is more likely to terminate in response to a painless therapy than if the value is outside the range, delivering the painless therapy to the patient; and if the value is outside the range, delivering a second therapy, comprising least one of defibrillation and electrical cardioversion, to the patient.

In some embodiments, the painless therapy comprises anti-tachycardia pacing. Some embodiments provide that the determining a value of a parameter and the delivering steps are performed by a device implanted in the patient. In some embodiments, the parameter comprises an indicator of depolarization of an intrinsic pacemaker in the patient's heart. Some embodiments provide that the intrinsic pacemaker comprises the sinoatrial node of the patient. Some embodiments provide that the parameter indicative of the rate comprises a sinus node cycle length.

In some embodiments, determining a value of a parameter comprises analyzing an atrial electrogram of the patient. Some embodiments provide that the determining a value of a parameter is performed using an electrode positioned in the patient's heart. In some embodiments, determining a value of a parameter is performed using an electrode positioned in an atrium of the patient.

Some embodiments provide that the second therapy of the above-mentioned embodiments comprises defibrillation. In some embodiments, the parameter comprises a sinus node cycle length and the determining a value of a parameter and the delivering steps are performed by a device implanted in the patient.

Some embodiments describe an implantable cardiac device or system, for treating a fast ventricular arrhythmia. In some embodiments, the device and/or system can include a determining module that determines a value of a parameter indicative of a rate of an intrinsic pacemaker of a heart of a patient experiencing a fast ventricular arrhythmia (FVA) and a delivery module, programmed to deliver a first therapy for terminating the FVA to the patient if the value indicates the rate is about equal to or higher than a threshold, and to deliver a second therapy for terminating the FVA, different from the first therapy, to the patient if the value indicates the rate is lower than the threshold.

In some embodiments, the rate is a depolarization rate. In some embodiments, the parameter is indicative of a rate of the sinoatrial node of the patent. Some embodiments provide that the parameter comprises a sinus node cycle length. In some embodiments, at least one of the first and second therapies is electrical. In some embodiments, the determining module analyzes an atrial electrogram of the patient, and in some embodiments, the determining module uses information derived from a signal transmitted via an electrode, positioned in an atrium of the patient, to determine the parameter value.

Some embodiments of the device and/or system provide that the first therapy comprises anti-tachycardia pacing, and some embodiments provide that the second therapy comprises at least one of defibrillation and electrical cardioversion. In some embodiments, the second therapy comprises defibrillation. In certain embodiments, the first therapy comprises anti-tachycardia pacing, and the second therapy comprises defibrillation.

In certain examples, a method, of treating a fast ventricular arrhythmia, is described including determining a value of a parameter indicative of a rate of an intrinsic pacemaker of a heart of a patient experiencing a fast ventricular arrhythmia (FVA); if the value indicates the rate is about equal to or higher than a threshold, delivering an electrical therapy, having a first value of a therapy parameter, to the patient for terminating the FVA; and if the value indicates the rate is lower than the threshold, delivering the electrical therapy, having a second value of the therapy parameter, to the patient.

In some embodiments, the rate is a depolarization rate. In some embodiments, the therapy parameter comprises at least one of a pulse waveform, a pulse rate, a pulse amplitude, a pulse width, and a pulse interval. In certain embodiments, the intrinsic pacemaker comprises the sinoatrial node of the patient. In some embodiments, the parameter indicative of the rate comprises a sinus node cycle length. Some embodiments provide that the fast ventricular arrhythmia comprises ventricular tachycardia. In some embodiments, the ventricular tachycardia has a cycle length equal to or less than about 240 ms.

In some embodiments, determining a value of a parameter indicative of a rate of an intrinsic pacemaker comprises analyzing an atrial electrogram of the patient. In certain embodiments, determining a value of a parameter indicative of a rate of an intrinsic pacemaker is performed using an electrode positioned in the patient's heart. Some embodiments provide that determining a value of a parameter indicative of a rate of an intrinsic pacemaker is performed using an electrode positioned in an atrium of the patient.

In some embodiments, the first therapy comprises a therapy that is relatively painless to the patient. Some embodiments provide that the first therapy comprises anti-tachycardia pacing. In certain embodiments, the second therapy comprises at least one of defibrillation and electrical cardioversion. In some embodiments, the second therapy comprises defibrillation. In certain embodiments, the first therapy comprises anti-tachycardia pacing, and the second therapy comprises defibrillation. Some embodiments provide that determining a value of a parameter indicative of a rate of an intrinsic pacemaker and the delivering steps are performed by a device implanted in the patient.

Some examples described herein relate to a method, of treating a ventricular arrhythmia, that includes determining a change in a sinus node cycle length of a heart of a patient between a time prior to the ventricular arrhythmia and a time during the ventricular arrhythmia; when the change in the sinus node cycle length is within a first range, delivering a first therapy to the patient for treating the ventricular arrhythmia; and when the change in the sinus node cycle length is within a second range, delivering a second therapy to the patient for treating the ventricular arrhythmia, wherein the first therapy is different from the second therapy.

In some embodiments, the change within the first range corresponds to lengthening of the sinus node cycle length during the ventricular arrhythmia. In some embodiments, the change within the second range corresponds to shortening of the sinus node cycle length during the ventricular arrhythmia.

In some embodiments, the first therapy comprises at least one of defibrillation and electrical cardioversion, and the second therapy comprises anti-tachycardia pacing. In some embodiments, the first therapy comprises a first number of anti-tachycardia pacing (ATP) attempts, and the second therapy comprises a second number of ATP attempts, the second number being greater than the first number. In some embodiments, each of the ATP attempts comprises a sequence of pulses delivered to one or both ventricles of the patient. In some embodiments, the determining and the delivering are performed by a device implanted in the patient.

In some embodiments, the determining the change in the sinus node cycle length comprises determining a first mean sinus node cycle length within a first time window prior to the onset of the ventricular arrhythmia; determining a second mean sinus node cycle length within a second time window from the onset of the ventricular arrhythmia; and determining the change in the sinus node cycle length based on the first and second mean sinus node cycle lengths.

Some examples provide a method, of treating an abnormal heart rhythm, that includes determining a change in a parameter indicative of a sinus node cycle length of a heart of a patient between a time prior to the abnormal heart rhythm and a time during the abnormal heart rhythm; when the change in the parameter indicative of the sinus node cycle length is within a first range, delivering a first therapy to the patient for treating the abnormal heart rhythm; and when the change in the parameter indicative of the sinus node cycle length is within a second range, delivering a second therapy to the patient for treating the abnormal heart rhythm, wherein the first therapy is different from the second therapy.

In some embodiments, the change within the first range corresponds to lengthening of the sinus node cycle length during the abnormal heart rhythm. In some embodiments, the change within the second range corresponds a shortening of the sinus node cycle length during the abnormal heart rhythm. In some embodiments, the first therapy comprises at least one of defibrillation and electrical cardioversion, and the second therapy comprises anti-tachycardia pacing. In some embodiments, the first therapy comprises a first number of anti-tachycardia pacing (ATP) attempts, and the second therapy comprises a second number of ATP attempts, the second number being greater than the first number. In some embodiments, the determining and the delivering are performed by a device implanted in the patient.

Some embodiments provide an implantable cardiac device, for treating an abnormal heart rhythm, that includes a determining module that determines a change in a parameter indicative of a sinus node cycle length of a heart of a patient between a time prior to the abnormal heart rhythm and a time during the abnormal heart rhythm; and a delivery module, programmed to deliver a first therapy for treating the abnormal heart rhythm to the patient when the change in the parameter is within a first range, and to deliver a second therapy for treating the abnormal heart rhythm, different from the first therapy, to the patient when the change in the parameter is within a second range.

In some embodiments, the change within the first range corresponds to lengthening of the sinus node cycle length during the abnormal heart rhythm. In some embodiments, the change within the second range corresponds to shortening of the sinus node cycle length during the abnormal heart rhythm. In some embodiments, the first therapy comprises at least one of defibrillation and electrical cardioversion, and the second therapy comprises anti-tachycardia pacing. In some embodiments, the first therapy comprises a first number of anti-tachycardia pacing (ATP) attempts, and the second therapy comprises a second number of ATP attempts, the second number being greater than the first number.

Some examples provide a method, of treating an abnormal heart rhythm, that includes determining a change in a parameter indicative of a sinus node cycle length of a heart of a patient between a time prior to the abnormal heart rhythm and a time during the abnormal heart rhythm; and selecting, based on the change in the parameter indicative of the sinus node cycle length, at least one of a first therapy and a second therapy for treatment of the abnormal heart rhythm.

Some embodiments provide an implantable cardiac device, for treating an abnormal heart rhythm, that includes: a determining module that determines a change in a parameter indicative of a sinus node cycle length of a heart of a patient between a time prior to an abnormal heart rhythm and a time during the abnormal heart rhythm; and a delivery module programmed to apply, based on the change in the parameter, at least one of a first therapy and a second therapy, different from the first therapy, for treatment of the abnormal heart rhythm.

Some embodiments provide an implantable cardiac device, for treating an abnormal heart rhythm, that includes: a determining module that determines a change in a parameter indicative of a sinus node cycle length of a heart of a patient between a time prior to an abnormal heart rhythm and a time during the abnormal heart rhythm; and a delivery module programmed to apply, based on the change in the parameter, at least one of a first therapy and a second therapy for treatment of the abnormal heart rhythm.

In some embodiments, the determining module determines a first mean sinus node cycle length within a first time window prior to the onset of the abnormal heart rhythm, determines a second mean sinus node cycle length within a second time window after the onset of the abnormal heart rhythm, and determines the change in the sinus node cycle length based on the first and second mean sinus node cycle lengths.

In some embodiments, the first therapy comprises at least one of anti-tachycardia pacing, defibrillation, and electrical cardioversion, and the second therapy comprises anti-tachycardia pacing. In certain embodiments, when the first therapy comprises a first number of anti-tachycardia pacing attempts, the second therapy comprises a second number of anti-tachycardia pacing attempts, the second number being greater than the first number. In some embodiments, each of the anti-tachycardia pacing attempts comprises a sequence of pulses delivered to a ventricle of the heart.

In some embodiments, the delivery module is further programmed to deliver the first therapy to the patient when the change in the parameter is within a first range, and to deliver the second therapy to the patient when the change in the parameter is within a second range. In some embodiments, the first range corresponds to a lengthening of the sinus node cycle length during the abnormal heart rhythm. In some embodiments, when the change corresponds to a lengthening of the sinus node cycle length, the delivery module applies, based on a sinus node cycle length after the onset of the abnormal heart rhythm, at least one of anti-tachycardia pacing, defibrillation, and electrical cardioversion. In certain embodiments, the delivery module applies at least one of defibrillation and electrical cardioversion when the sinus node cycle length after the onset of the abnormal heart rhythm is less than or equal to about 240 ms. In some embodiments, the delivery module applies anti-tachycardia pacing when the sinus node cycle length after the onset of the abnormal heart rhythm is greater than about 240 ms.

In some embodiments, the second range corresponds to a shortening of the sinus node cycle length during the abnormal heart rhythm. In some embodiments, when the change corresponds to a shortening of the sinus node cycle length, the delivery module applies anti-tachycardia pacing. In some embodiments, the delivery module repeats the anti-tachycardia pacing based on a sinus node cycle length after the onset of the abnormal heart rhythm.

For purposes of summarizing the disclosure, certain aspects, advantages, and novel features of the disclosure have been described herein. It is to be understood that not necessarily all such advantages may be achieved in accordance with any particular embodiment of the disclosure. Thus, the disclosure may be embodied or carried out in a manner that achieves or optimizes one advantage or group of advantages as taught herein without necessarily achieving other advantages as may be taught or suggested herein.

### Brief Description of the Drawings

General descriptions provided herein that implement various features of the disclosure will now be described with reference to the drawings. The drawings and the associated descriptions are provided to illustrate embodiments of the disclosure and not to limit the scope of the disclosure.
Figure 1 depicts sample tracings of integrated sympathetic neurogram, arterial blood pressure, central venous pressure, and electrocardiogram during baseline and during rapid pacing in accordance with embodiments described herein.
Figure 2A depicts changes in mean blood (arterial) pressure (MBP, MAP) during pacing, with near simultaneous atrial and ventricular systole (A&V), short-RP tachycardia, and long-RP tachycardia.
Figure 2B depicts changes in central venous pressure (CVP) during pacing, with near simultaneous atrial and ventricular systole (A&V), short-RP tachycardia, and long-RP tachycardia.
Figure 2C depicts changes in sympathetic nerve activity (SNA) during pacing, with near simultaneous atrial and ventricular systole (A&V), short-RP tachycardia, and long-RP tachycardia.
Figure 3A depicts changes in MAP in response to pacing and nitroprusside infusion (NTP).
Figure 3B depicts changes in CVP in response to pacing and nitroprusside infusion (NTP).
Figure 3C depicts changes in SNA in response to pacing and nitroprusside infusion (NTP).
Figure 4 depicts a chart comparing changes in MAP and SNA in response to pacing and nitroprusside infusion (NTP).
Figure 5A depicts changes in MAP in response to ventricular pacing (VP) and VP with phenylephrine infusion (PE) or head-up tilt (HUT).
Figure 5B depicts changes in pulmonary arterial pressure (PAP) in response to ventricular pacing (VP) and VP with phenylephrine infusion (PE) or head-up tilt (HUT).
Figure 5C depicts changes in SNA in response to ventricular pacing (VP) and VP with phenylephrine infusion (PE) or head-up tilt (HUT).
Figure 6 depicts a relationship of recovery of MAP during VP to arterial baroreflex sympathetic gain estimated during NTP infusion.
Figure 7 depicts an upper depicting normal sinus rhythm with a sinus node cycle length (SNCL) equal to about 747 ms and a lower panel during VF, the mean SNCL decreased to 659 ms.
Figure 8A depicts a normal sinus rhythm with a mean SNCL equal to about 651 ms.
Figure 8B depicts, during VF, the mean SNCL having increased to about 688 ms.
Figure 9 depicts the percent change in SNCL from baseline at baseline and following the administration of placebo, atropine (A), propranolol (P) and atropine in addition to propranolol (A+P).
Figure 10A depicts results of changing SNCL, compared to VF induction (NSR), and during a 30-second period of VF, in which the SNCL decreased.
Figure 10B depicts results of changing SNCL, compared to NSR, and during a 30-second period of VF, in which the SNCL increased.
Figure 11A depicts ECG recordings during VF induction in a patient undergoing an ICD implant.
Figure 11B depicts BP recordings during VF induction in a patient undergoing an ICD implant.
Figure 11C depicts SNA recordings during VF induction in a patient undergoing an ICD implant.
Figure 12 depicts changes in MBP when compared to NSR during 10-second increments of a 30-second period of VF.
Figure 13 depicts a determining module in connection with a delivery module, in accordance with embodiments described herein.
Figure 14 is a flowchart showing the operation of an implantable cardiac device for treating ventricular arrhythmia according to an embodiment of the present invention
Figure 15 is a flowchart of an exemplary method for treating an abnormal heart rhythm.

### Detailed description

The present disclosure provides systems and methods for treating cardiac arrhythmias. Some embodiments include an implantable cardiac device or system, for treating a fast ventricular arrhythmia. Certain embodiments can include a determining module that determines a value of a parameter indicative of a rate of an intrinsic pacemaker of a heart of a patient experiencing FVA. In some embodiments, for example, the rate can be a depolarization rate. Certain embodiments can include a delivery module, programmed to deliver a plurality of therapies to a patient experiences cardiac arrhythmia. A first therapy can be delivered, for terminating the FVA, to the patient if the value indicates the rate is about equal to or higher than a threshold. A second therapy, different from the first therapy, can be delivered to the patient if the value indicates the rate is lower than the threshold.

Some examples relate to a method of treating a fast ventricular arrhythmia that includes the step of determining a value of a parameter indicative of a rate of an intrinsic pacemaker of a heart of a patient experiencing a fast ventricular arrhythmia (FVA). In some embodiments, the rate is a depolarization rate. The method can provide treatment for a patient based on whether the rate is within a threshold or beyond such threshold. If the value indicates the rate is about equal to or higher than the threshold, the method provides for delivering a first therapy to the patient for terminating the FVA. If the value indicates the rate is lower than the threshold, the method provides for delivering a second therapy, different from the first therapy, to the patient for terminating the FVA.

In some embodiments, if the value indicates the rate is lower than a threshold, some methods provide for delivering a third therapy that stimulates the patient's sympathetic nervous system. In certain embodiments, the third therapy is sufficient to raise an arterial blood pressure in the patient. Some embodiments provide that the third therapy is electrical. In some embodiments, the third therapy comprises delivery of a sympathetic or sympathomimetic agent to the patient. In some embodiments, the third therapy is a combination of electrical and delivery of a sympathetic or sympathomimetic agent to the patient. In some embodiments, the agent may be delivered through an electrode that may be used for diagnostic or therapeutic purposes. Examples of additional drug delivery methods are know in the art and may be implemented accordingly. Examples of agents that can be used include epinephrine, phenylephrine, dopamine, etc.

In most circumstances, the application of painless therapy to fast ventricular arrhythmias (FVA; e.g., Cycle Length (CL)<240ms) is limited by the inability to predict who is able to tolerate the arrhythmia without loss of consciousness. Described herein are tools to predict blood pressure response during FVA, thus enabling the application of painless therapy in patients who can tolerate it.

During simulated ventricular tachycardia (e.g., CL 240ms), the arterial baroreflex plays a major role in mediating the sympathetic changes with minimal contribution from the cardiopulmonary baroreflex (Circulation. 1999 Aug. 10;100(6):628-34 (incorporated herein by reference)). Arterial baroreflex gain correlates with the blood pressure response (Circulation. 1999 Jul 27;100(4):381-6 (incorporated herein by reference)). In follow-up studies aimed at evaluating the role of the baroreflex in ventricular fibrillation (e.g., CL<240ms), no correlations were found between arterial baroreflex gain and the changes in sinus node cycle length (SNCL). SNCL did not change and even lengthened in some patients, suggesting vagal activation and/or sympathoinhibition.

It has been observed that peripheral sympathetic activity decreased during induced ventricular fibrillation in a small number of patients. The lack of change, and even lengthening of the SNCL, combined with the finding of sympathoinhibition suggested that, in some patients, a "vasovagal"-like reaction could be taking place. During a vasovagal reaction, a paradoxical response occurs resulting in heart rate slowing, i.e., SNCL lengthening and sudden vasodilatation leading to a decrease in blood pressure. If this paradoxical response were to occur during FVA, the blood pressure response would be different in those with a vasovagal reaction when compared to those without a vasovagal reaction. By assessing the SNCL changes during the arrhythmia, it is possible to get an indication as to who will have a greater decrease in blood pressure and thus potentially an earlier loss of consciousness. The application of painless therapy in patients who develop of a vasovagal like reaction might be dangerous since they are more prone to lose consciousness earlier than those who do not develop a vasovagal like reaction. However, its use in patients without a vasovagal reaction might be beneficial, in that additionally treatment methodologies can be implemented. In some of these patients, successful painless therapy will prevent the delivery of painful shocks, thus improving the quality of life of thousands of patients with implantable defibrillators in addition to increasing battery longevity.

In conducting analysis of the possible "vasovagal" reaction it was found that: 1) SNCL did not change and even lengthened in a small number of animals; 2) the mechanism of the SNCL changes appeared to be vagally mediated; and 3) the percent decrease in blood pressure was greater in the animals with no SNCL changes or SNCL lengthening when compared to those with SNCL shortening. These findings were made from a limited number of studies.

It is also possible to distinguish the blood pressure response in patients without vasovagal reaction, i.e., those with SNCL shortening, by assessing the magnitude of the change in SNCL. The greater the SNCL shortening, the lesser the decrease in blood pressure, thus further improving the algorithm for painless therapy. No algorithm in place uses a device data to predict blood pressure response during ventricular arrhythmias. This disclosure will highlight what information should be looked at, and more importantly, will provide guidelines on how to use it in thousands of patients with devices.

Shock therapy has a major impact on the quality of life of thousands of patients with implantable defibrillators. While painless therapy has been shown to be effective in the treatment of ventricular arrhythmias with, for example, CL greater than about 240 ms, its application in the treatment of rapid ventricular arrhythmias with, for example, CL less than about 240ms has been limited. The major concern has been the incidence of syncope. Proposed herein are new methods and systems that enable the identification of patients who can better tolerate painless therapy.

Simulated VT is associated with an increase in sympathetic nerve activity (SNA), and the magnitude of sympathoexcitation is correlated with arterial baroreflex gain. SNA recordings have not previously been obtained in patients during VF. In addition, the relationship between sinus node cycle length (SNCL) changes, and SNA during VF has not been evaluated.

In some methods, patients receiving dual chamber ICD implants with defibrillation threshold testing were asked to enroll. SNA recordings were attempted in all patients. In addition, the mean SNCL was measured during the 5 seconds preceding VF onset and the last 5 seconds before defibrillation. Seven patients were enrolled, and SNA measurements were successfully obtained in 3 patients. Atrial recordings were available in 6 patients. In patients with successful SNA recordings, sympathoinhibition was observed during VF in 2 patients, with sympathoexcitation noted in the remaining patient. Compared to baseline, SNCL shortened in 4 patients (-14% from baseline) and lengthened in the remaining 2 patients (+2% from baseline). In those with successful SNA recordings and SNCL measurements, the increase and decrease in SNA was associated with a reduction and lengthening in SNCL. These findings of sympathoinhibition associated with SNCL lengthening suggest the presence of vasovagal-like physiology during VF in a subgroup of patients. The implications regarding the magnitude of BP fall and application of painless therapy remain to be evaluated.

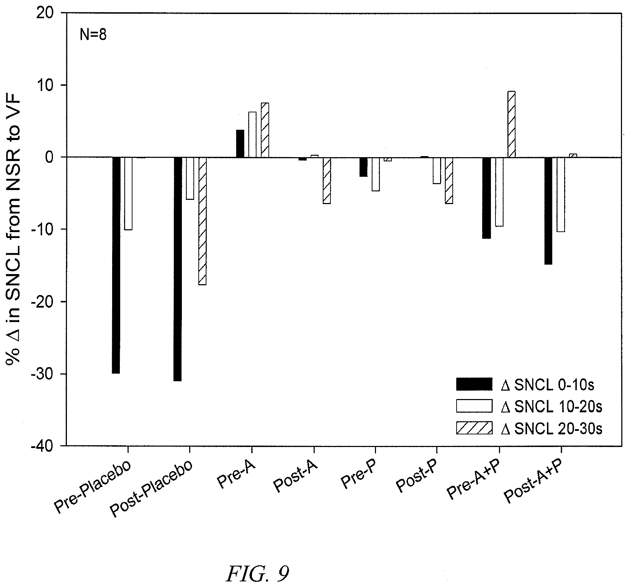

It has been shown in humans and in a porcine model that sinus node cycle length (SNCL) increases during VF in a significant number of cases, although it is uncertain what mechanism is responsible for the SNCL changes during VF. In eight anesthetized pigs, the chest was opened and the heart was exposed on a pericardial cradle. A 247-electrode sock was placed around the ventricles, an atrial lead over the right atrial appendage and an arterial catheter in the right femoral artery for blood pressure monitoring. Using DC current or the shock-on-T method, VF was induced and sustained for 30 seconds before applying a DC shock. Following a 10 minute recovery period, atropine (A, n=2) at 0.04mg/kg, propranolol (P, n=2) at 0.2mg/kg, both (A+P, n=2) or neither (Placebo, n=2) were administered at random, and VF was re-induced. SNCL changes were assessed before and after drug/placebo administration. Percent SNCL change (%ΔSNCL) was defined as %ΔSNCL=(VF-SNCL-Baseline-SNCL)/(Baseline-SNCL)*100. Atropine administration reversed the SNCL lengthening observed during VF while propranolol had little or no effect on SNCL shortening (see Fig. 9). The SNCL changes during the first 30 seconds of VF appear to be vagally mediated. The relationship between SNCL changes and peripheral sympathetic activity remain uncertain.

It has been shown that sinus node cycle length (SNCL) increases during VF in a significant number of cases. The relationship between SNCL changes and systemic BP remain unknown. In specific, whether SNCL lengthening is associated with peripheral sympathoinhibition and a greater decrease in BP, i.e., a vasovagal-like reaction is unclear. In test involving eleven anesthetized pigs, the chest was opened and the heart was exposed on a pericardial cradle. A 247-electrode sock was placed around the ventricles, an atrial lead over the right atrial appendage and an arterial catheter in the right femoral artery for blood pressure monitoring. Using DC current or the shock-on-T method, VF was induced and sustained for 30 seconds before applying a DC shock. Animals were divided in to 2 groups based on SNCL response during VF: Shortening group (n=7, %ΔSNCL= -17%), Lengthening group (n=4, %ΔSNCL=4%). RESULTS: In the Shortening group, mean BP fell by 53% in the first 10 sec. when compared to NSR and continued to fall to 64% and 68% at 20 sec. and 30 sec., respectively. In the Lengthening group, BP fell by 72%, 81% and 83% when compared to baseline. The differences in percent decrease in BP between the groups were statistically significant at all 3 time points during VF (p<0.05). SNCL lengthening was associated with a greater decrease in BP when compared to SNCL shortening. The above findings suggest that SNCL changes during VF might be helpful in predicting the magnitude of the hemodynamic fall in BP. Such information could be useful in deciding who has "time" for painless therapy before delivering shock therapy in patients with dual chamber ICDs.

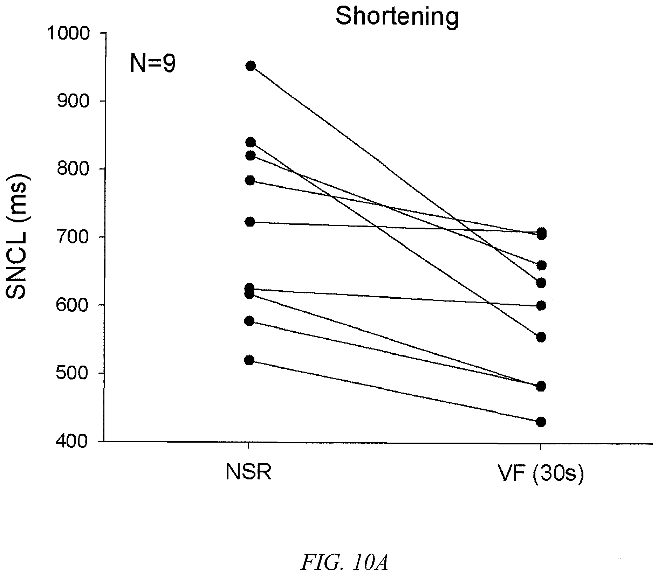

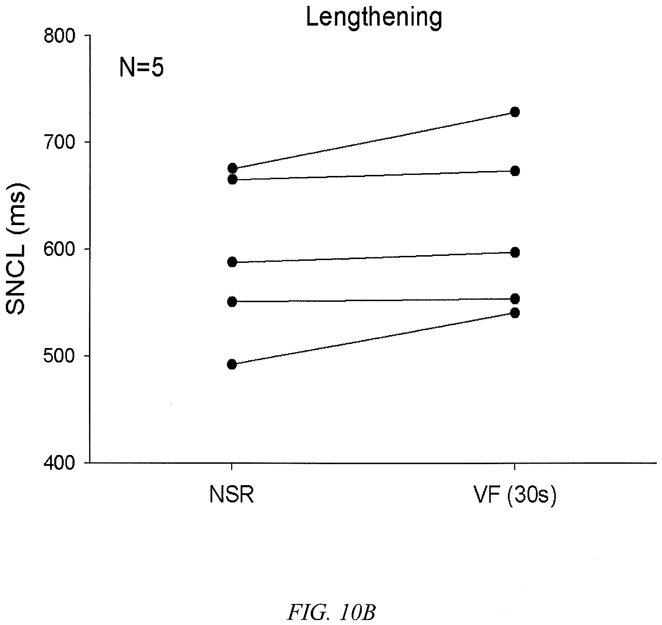

While some studies have evaluated the autonomic changes prior to VF onset, the changes during VF remain poorly understood. Analysis of the SNCL changes during VF provides a unique opportunity to assess the autonomic changes. In fourteen anesthetized pigs, the chest was opened and the heart was exposed on a pericardial cradle. A 247-electrode sock was placed around the ventricles, an atrial lead over the right atrial appendage and an arterial catheter in the right femoral artery for blood pressure monitoring. Using DC current or the shock-on-T method, VF was induced and sustained for 30 seconds before applying DC shock. Mean SNCL was measured during the 10-second period prior to VF induction (NSR) and during the 30-second period of VF. Compared to NSR, SNCL during VF shortened in 9 animals and lengthened in 5 (36%), (see Figs. 10A and 10B). As a group, the percent change in SNCL during the first 10-sec interval was greater than the percent change during the second and third 10-sec. intervals. Similarly, the percent change during the second 10-sec. interval was greater than the percent change in the third 10-sec interval. SNCL lengthening was observed in 36% of the pigs during the first 30 seconds of VF suggesting increased vagal tone or decreased sympathetic activity in one third of the cases. In addition, there was a gradual increase in SNCL regardless of the magnitude or direction of the initial change. The underlying mechanism of the SNCL changes and the clinical implications remain unknown.

The role of the baroreflex system in various tachyarrhythmias has been the subject of extensive research. During tachyarrhythmias, arterial blood pressure (BP) decreases while central venous pressure (CVP) increases. On one hand, the unloading of the arterial baroreceptors results in an increase in sympathetic nerve activity (SNA) and vagal inhibition via the arterial baroreflex. On the other hand, the increase in filling pressures results in a decrease in SNA and an increase in vagal tone via the cardiopulmonary baroreflex. These competing reflexes result in mixed messages to the central nervous system. It has been shown that during supraventricular and ventricular tachycardia, the arterial baroreflex predominates with minimal contribution from the cardiopulmonary reflex. Indeed, the net response during these tachycarrhythmias is a state of sympathoexcitation, which has been shown to correlate with blood pressure recovery. While the autonomic changes during supraventricular and ventricular tachycardia have been described, the responses during fast ventricular arrhythmias (FVA: VF and rapid VT with about CL<240ms) remain unknown.

Assessment of the autonomic changes during ventricular arrhythmias is a challenge as the surface ECG prohibits the evaluation of sinus node function. Thus far, human studies have used microneurography as the only direct method for assessing the sympathetic changes during tachyarrhythmias. With the increased number of defibrillator implants incorporating atrial leads, analysis of the changes in sinus node cycle length (SNCL) from atrial electrograms recorded provides a unique opportunity to assess the autonomic changes that accompany these arrhythmias. We have recently shown in defibrillator patients that SNCL shortening during ventricular tachycardia, a marker of the degree of sympathoexcitation, was a predictor of successful anti-tachycardia pacing. Reflex sympathoexcitation is known to improve conduction and shorten ventricular refractory period, thus increasing the likelihood that a pacing impulse reaches the excitable gap and collides both orthodromically and antidromically with the tachycardia wavefront. In another study, we assessed whether the changes in SNCL during VF correlated with arterial baroreflex gain (BRG). BRG and SNCL measurements were successfully obtained in 18 patients undergoing the implantation of an implantable cardiovertor defibrillator (ICD). During VF, SNCL shortened in 11 patients and surprisingly lengthened in 7 patients. We found no correlation between arterial BRG and percent change in SNCL. To our knowledge, the mechanisms underlying the SNCL changes during VF and VT with CL<240ms remain unknown.

Determination of the mechanisms responsible for the SNCL changes during FVA (VF and VT with about CL< 240ms) and its relationship to peripheral sympathetic activity would be advantageous. It is believed the changes in SNCL during FVA are vagally mediated, and that the lengthening and shortening in SNCL are associated with a decrease and an increase in peripheral sympathetic activity respectively. Accordingly, it is understood that some patients develop paradoxically a vasovagal-like reaction during FVA, and the analysis of the SNCL could help identify this subgroup of patients.

Increased sympathetic activity during ventricular tachycardia has been shown to be beneficial. Indeed, our group has shown that the greater the sympathoexcitation was during ventricular tachycardia, the greater was the BP recovery both during and after tachycardia termination.

It is believed that the effects of FVA (VF and VT with about CL<240ms) on peripheral sympathetic activity have not been evaluated. In addition, the role of the autonomic changes in mediating blood response and time to symptoms remain unknown. The study described above was the first to look at the sinus rate as a surrogate of the autonomic changes that occur during VF. If the lengthening and shortening in SNCL are indeed associated with a decrease and an increase in peripheral sympathetic activity respectively, then patients with SNCL lengthening should have a greater decrease in BP due to a decrease in arteriolar resistance, and earlier onset of symptoms when compared to patients with SNCL shortening. Conversely, patients with SNCL shortening would have a lesser decrease in BP due to elevated arteriolar resistance and delayed onset of symptoms when compared to patients with SNCL lengthening.

Determination of the role of the autonomic changes during FVA in predicting blood pressure response and time to symptoms would be advantageous. It is believed that the SNCL lengthening during FVA is associated with a greater decrease in blood pressure when compared to those with SNCL shortening. Furthermore, it is believed that patients with SNCL lengthening have a shorter time to near syncope and syncope when compared to those with SNCL shortening.

The number of implantable cardioverter-defibrillators (ICD) implants per year, at the time of this disclosure, about 50,000 and is expected to grow as an aging population and expanding ICD indications intersect. A sub-study of the MADIT II ICD recipients revealed that, over an average of 17.2 months of follow-up, 24% of ICD recipients received an appropriate ICD therapy. Some ICDs, have a variety of programmable options for antitachycardia pacing (ATP), which can be an effective alternative to shocks. Despite encouraging results from studies employing ATP algorithms, the clinical predictors of successful ATP remain poorly understood. Furthermore, this therapy is rarely used for the treatment of fast ventricular arrhythmias (CL<240ms) because of the fear of syncope should ATP fails. Therefore, gaining an insight about the BP response and time to symptoms onset during FVA should greatly enhance our utilization of this painless therapy. Indeed, ICD shocks have shown to be associated with newly diagnosed depression and anxiety disorders in addition to their impact on battery life longevity. Therefore, any decrease in the number of shocks through the utilization of successful ATP should have a great impact on the quality of life of millions of Americans with ICD implants in addition to device longevity.

Information derived from the studies discussed above are incorporated into an algorithm that uses ATP therapy in patients with FVA. As stated above, ATP is rarely used for the treatment of FVA (CL<240ms) because of the fear of syncope should ATP fails. It is believed that the SNCL changes can provide further information on the early BP response and time to symptoms in patients with FVA. In specific, it is believed that anti-tachycardia pacing reduces shock therapy for fast ventricular arrhythmias in patients with SNCL shortening without a significant increase in acceleration or syncope.

Supraventricular tachycardias (SVT) with a 1:1 atrioventricular (AV) relationship are classified according to the location of the P wave in relation to the QRS: Short RP tachycardias (RP<PR), long RP (RP>PR) tachycardias and tachycardias with no identifiable P waves. Little is known about the significance of the P wave relation to the QRS in terms of hemodynamic and neural outcome. The effect of atrial systole timing on the hemodynamic and sympathetic neural response during rapid dual chamber pacing (DDD) was evaluated in 10 patients with a DDD pacemaker. Blood pressure, central venous pressure and sympathetic nerve activity (SNA) were recorded continuously during rapid DDD pacing at a rate of 175 bpm (CL=342 ms) with 3 AV intervals: AV= 30 ms, AV= 200 ms and AV= 110 ms, simulating SVT with no identifiable P wave, short RP and long RP tachycardia respectively. The relationship of atrial systole to ventricular systole was found to play a major role in the hemodynamic and neural response during tachycardia, with long RP tachycardia having the most favorable response. The changes in SNA seem to parallel the changes in mean BP with no clear evidence of predominant atrial vasodepressor response.

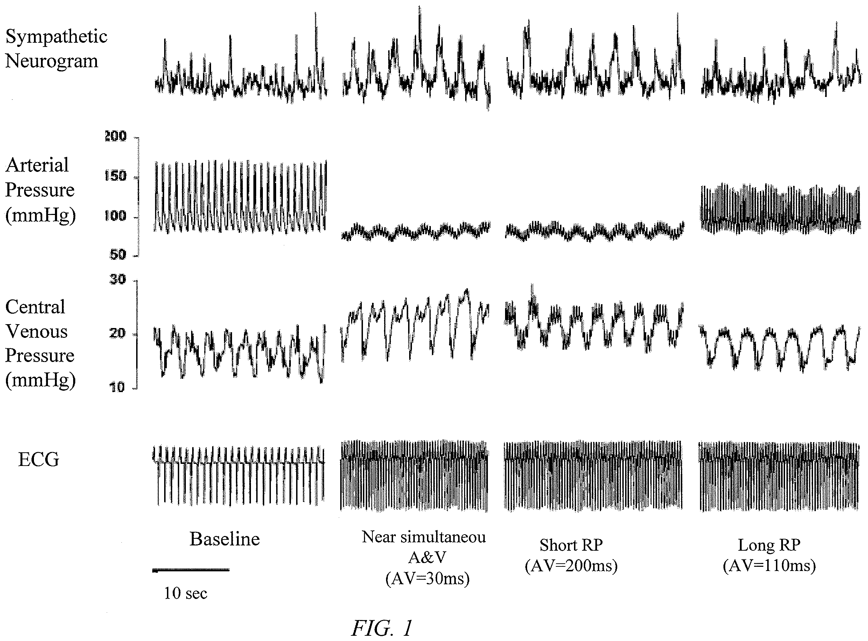

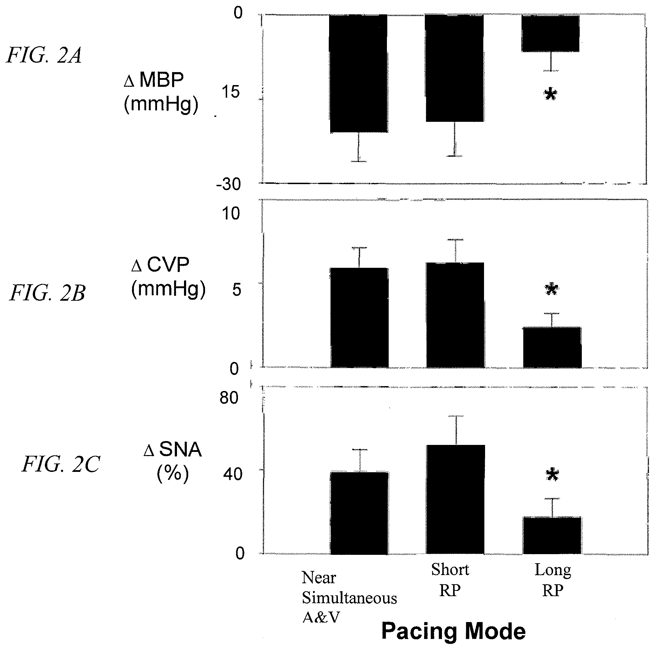

Fig. 1 depicts sample tracings of integrated sympathetic neurogram, arterial BP, CVP, and ECG during baseline (prepacing) and during minute 3 of rapid pacing (175 bpm) with either near-simultaneous atrial and ventricular systole (A&V), short-RP tachycardia, or long-RP tachycardia. In this individual, it is apparent that long-RP tachycardia produced a higher arterial BP, lower CVP, and less increase in SNA. Figs. 2A-2C depict changes (mean±SEM) in MAP (DMAP), CVP (DCVP), and SNA (DSNA) during minute 3 of pacing with near simultaneous atrial and ventricular systole (A&V), short-RP tachycardia, and long-RP tachycardia. The asterisks * in Figs. 2A-2C indicate a significant difference from the other 2 pacing modes (P<0.05).

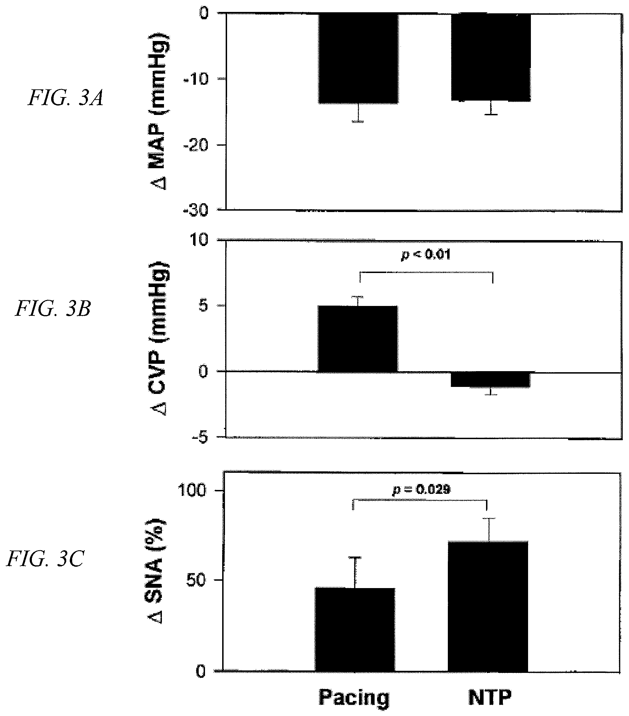

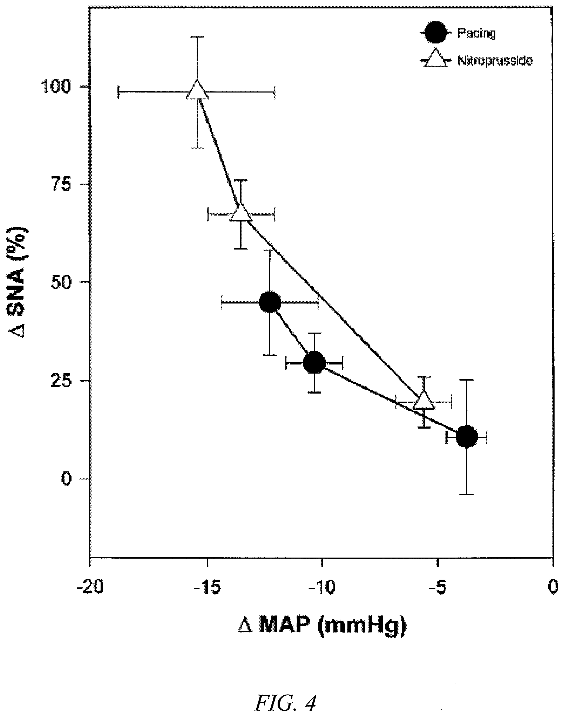

During ventricular tachycardia, BP decreases while CVP increases. The decrease in BP is expected to result in an increase in SNA due to unloading of the arterial baroreceptors, while the increase in CVP should result in a decrease in SNA due to activation of the cardiopulmonary baroreceptors. It is believed that arterial BRG predominated in mediating the sympathetic changes with minimal contribution from the cardiopulmonary baroreceptors. Furthermore, it is believed that arterial BRG correlated with the hemodynamic outcome during sustained ventricular tachycardia. In a test, efferent post-ganglionic muscle SNA, BP and CVP were measured during ventricular pacing (3 pacing CLs) and compared to the responses to 3 doses of nitroprusside infusion (NTP). We also measured SNA, BP and CVP during VP (400ms) under 3 conditions: 1) Pacing alone, 2) Pacing + Head-up tilt, and 3) Pacing + Phenylephrine. Figs. 3A-3C and Fig. 4 depict mean ± SEM responses to pacing and NTP that produced comparable decreases in MAP (13.6 ± 2.7 vs 13.2 ± 1.8 mmHg).

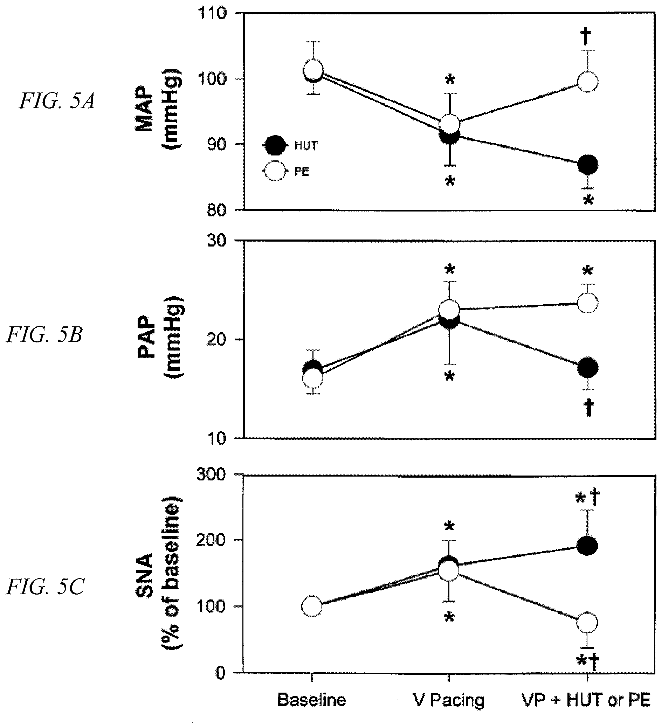

NTP resulted in a significantly greater increase in SNA when compared to VP (Figures 3A-3C and 4). The reason for that is that while both NTP and VP resulted in a decrease in BP and thus unloading of the arterial baroreceptors, NTP infusion was associated with a decreased in CVP while VP resulted in an increase in CVP. A decrease in CVP results in a greater increase in SNA due to unloading of the cardiopulmonary baroreceptors. On the other hand, an increase in CVP results in sympathoinhibition due to activation of the cardiopulmonary baroreceptors. The fact that VP resulted in an increase in SNA despite the increase in CVP suggests that arterial BRG predominates in mediating the SNA changes with minimal contribution form the cardiopulmonary BRG. This point is further illustrated in Figs. 5A-5C where head-up tilt added to VP resulted in a greater increase in SNA due to the associated reduction in CVP. On the other hand, phenylephrine infusion resulted in a decrease in SNA due to the increase in BP, which was the main trigger for the increase in SNA. Figs. 5A-5C depict summary data for MAP, PAP and SNA responses to ventricular pacing (VP) and VP with phenylephrine infusion (PE, ○) or head-up tilt (HUT, □). Mean ± SEM. The * indicates a significant difference from baseline (p<0.05), and the † indicates a significant difference from VP alone.

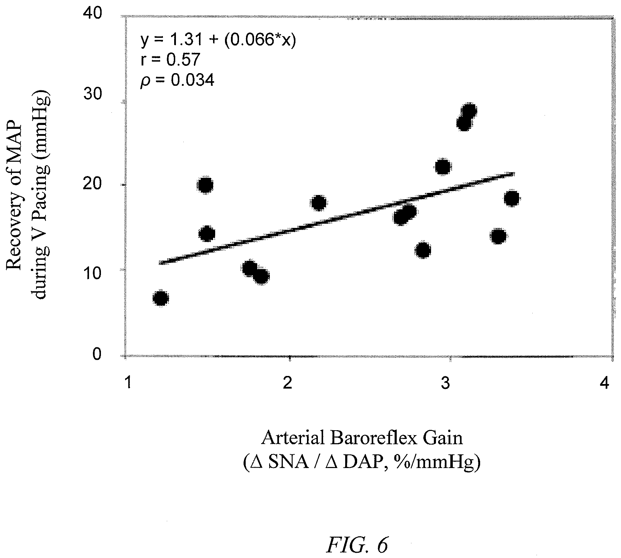

To test the proposal that arterial BRG was a predictor of hemodynamic outcome during VT, sympathetic nerve activity (SNA), BP and central venous pressure were measured in 14 patients during nitroprusside (NTP) infusion and during rapid VP, simulating VT. Arterial BRG was defined as the slope of the relationship of change in SNA (%) to change in diastolic BP during NTP. BP recovery during sustained VP was defined as the change in BP from the nadir to steady state during sustained pacing. We found that arterial BRG correlated positively with mean BP recovery (r=0.57) (See Fig. 6). Fig. 6 depicts the relationship of recovery of MAP during VP to arterial baroreflex sympathetic gain estimated during NTP infusion. Recovery of MAP was determined as a change in MAP from nadir at onset of pacing to steady-state level after 1 minute of pacing. Recovery of MAP correlated positively with arterial baroreflex gain (r=0.57, P-0.034) and diastolic arterial pressure is references as DAP.

While the above 2 studies evaluated the SNA changes during simulated SVT and VT, the SNA response during VF and rapid VT with CL<240 ms, remain unknown.

Analysis of the changes in sinus node cycle length (SNCL) during VF inductions in patients undergoing the implantation of a defibrillator incorporating an atrial lead provides a unique opportunity to assess the autonomic changes that accompany VF. The purpose of this study was to assess whether the arterial baroreflex, as measured by arterial baroreflex gain (BRG), was a predictor of the change in SNCL during VF and of BP recovery following successful defibrillation. We believed that SNCL changes during VF would correlate with arterial BRG, i.e., the greater the arterial BRG is, the greater the shortening in SNCL during VF. Arterial BRG was measured using the modified Oxford technique in 18 patients referred for the implantation of a defibrillator incorporating an atrial lead. The average SNCL was measured during the 5 seconds prior to VF induction and the last 5 seconds during VF before defibrillation. Percent SNCL change (%ΔSNCL) was determined.

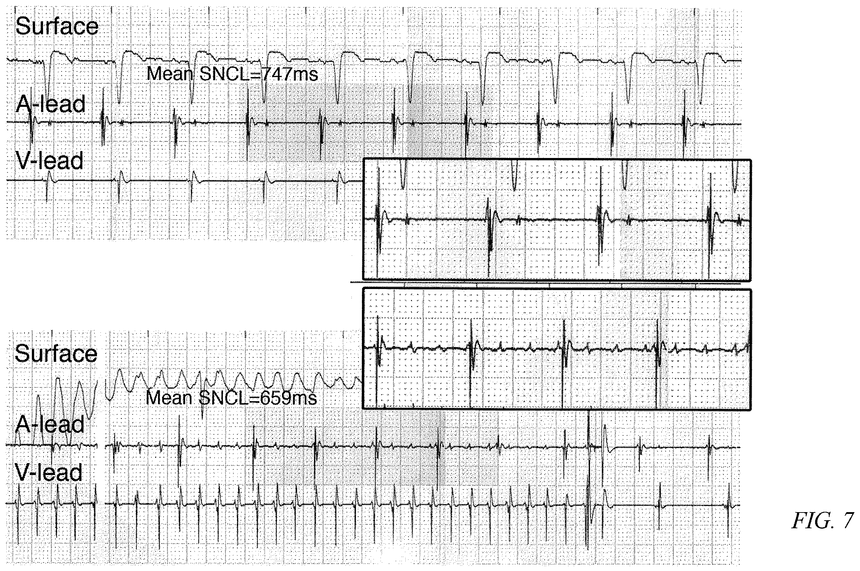

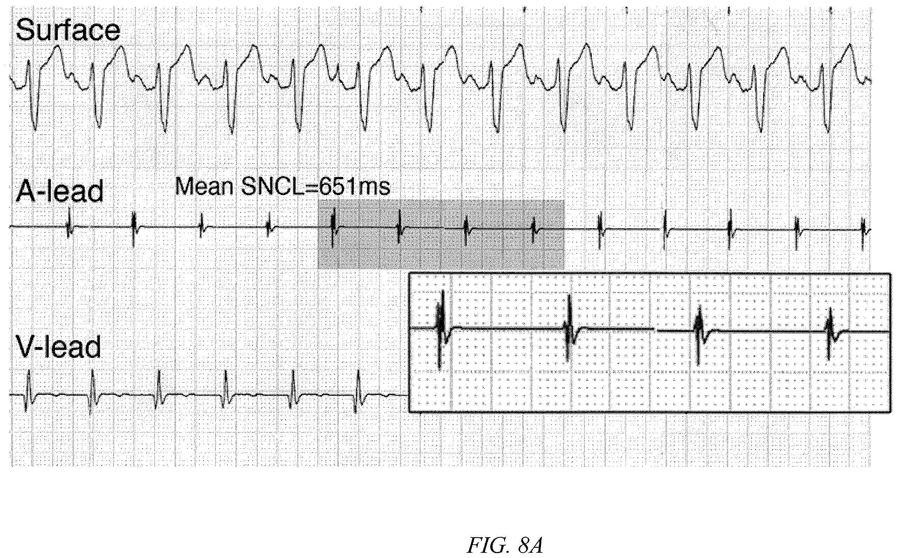

Arterial BRG ranged between -3 and 18 ms/mmHg. During VF, SNCL shortened in 11 patients (Group A, mean %ΔSNCL = -15%), and surprisingly lengthened in 7 patients (Group B, mean %ΔSNCL = 5%). There was no correlation between %ΔSNCL and arterial BRG gain (r = 0.25, p= 0.32). In fact, arterial BRG in Group A was lower when compared to Group B (p=0.075). Sample tracings showing SNCL shortening and lengthening during VF are provided in Figs. 7 and 8A-8B.

Fig. 7 depicts, in the upper panel, the normal sinus rhythm with a SNCL equal 747 ms. In the lower panel, Fig. 7 depicts, during VF, the mean SNCL decreased to 659 ms. For each panel, from top to bottom; surface ECG, atrial (A) signal, ventricular (V) signal, timing markers are illustrated, and the highlighted segment of the atrial signals are enlarged.

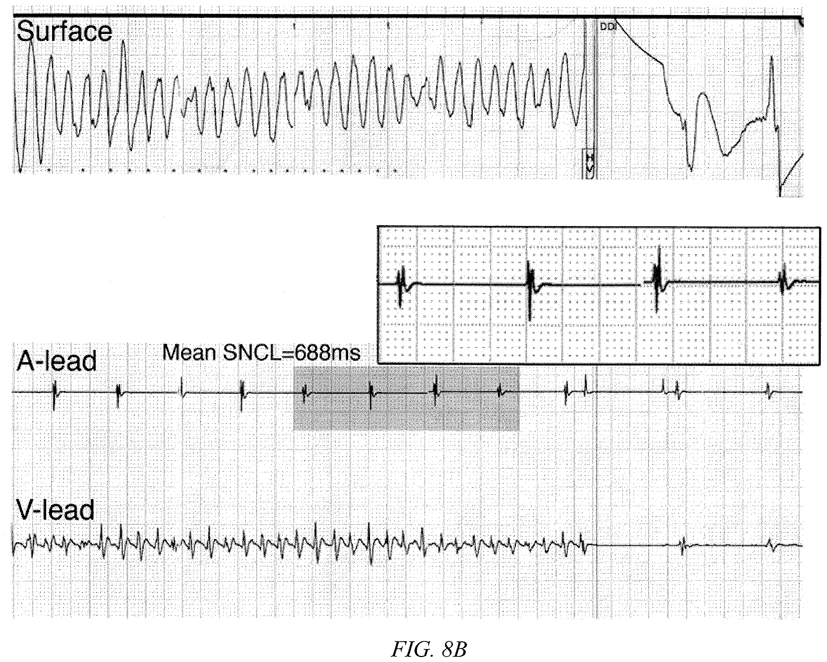

Fig. 8A depicts the normal sinus rhythm with a mean SNCL equal to 651 ms. Fig. 8B depicts, during VF, the mean SNCL did not decrease but rather increased to 688 ms. For each figure, from top to bottom, surface ECG, timing markers, atrial (A) signal, ventricular (V) signal are provided. The highlighted segments of the atrial signals are enlarged

This recent study highlights a significant new finding: SNCL lengthening during VF in almost 40% of the patients. The mechanisms underlying the SNCL changes and the clinical implications are unknown. Furthermore, whether the same findings occur with rapid VT with CL<240 ms remain unclear.

It is believed that, with respect to the mechanisms of SNCL changes during FVA, changes in SNCL during FVA are vagally mediated. A test was conducted in a swine model where SNCL changes will be measured during VF and RVP before and after selective parasympathetic, sympathetic and complete autonomic blockade.

In 8 anesthetized pigs, the chest was opened and the heart was exposed on a pericardial cradle. An atrial lead was placed over the right atrial appendage and an arterial catheter in the right femoral artery for BP monitoring. Using DC current or the shock-on-T method, VF was induced and sustained for 30 seconds before applying a DC shock. Following a 10 minute recovery period, atropine (A, n=2) at 0.04mg/kg, propranolol (P, n=2) at 0.2mg/kg, both (A+P, n=2) or neither (Placebo, n=2) were administered at random, and VF was re-induced. SNCL changes were assessed before and after drug/placebo administration. Percent SNCL change (%ΔSNCL) was defined as %ΔSNCL= (VF-SNCL - Baseline-SNCL)/(Baseline-SNCL)*100. Atropine administration reversed the SNCL lengthening observed in the 2 pigs while propranolol had no effect on SNCL shortening observed in the 2 other pigs. Fig. 9 depicts the %ΔSNCL at baseline and following the administration of placebo, atropine (A), propranolol (P) and atropine in addition to propranolol (A+P). Our findings suggest that the SNCL changes during the first 30 seconds of VF appear to be vagally mediated.

While the study above is assessing the mechanism of SNCL changes during FVA, the relationship between these changes and peripheral sympathetic activity remain unknown. It is believed that the lengthening and shortening in SNCL are associated with a decrease and an increase in peripheral sympathetic activity respectively. A test was conducted in patients undergoing the implantation of dual chamber ICDs or a generator change. Using the microneurography technique described above, muscle SNA and the SNCL changes during VF inductions and RVP were recorded.

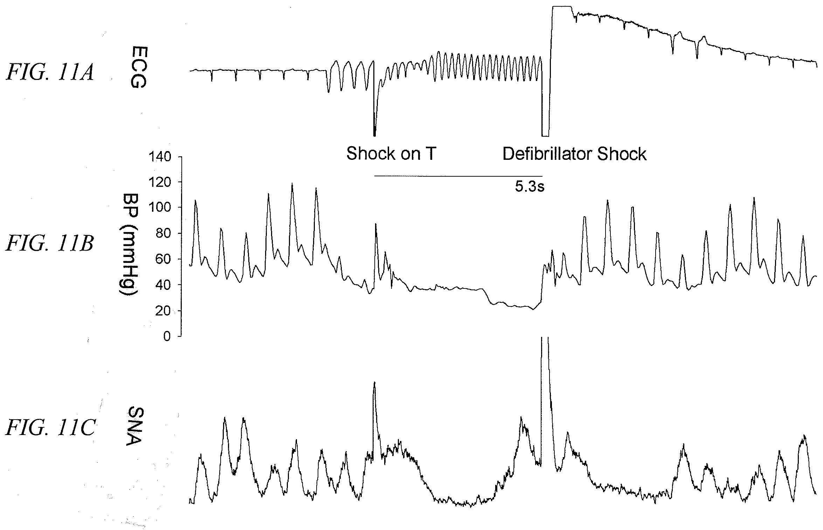

Patients receiving dual chamber ICD implants with defibrillation threshold testing were asked to enroll. SNA recordings were attempted in all patients. In addition, the mean SNCL was measured during the 5 seconds preceding VF onset and the last 5 seconds before defibrillation. Successful SNA recordings were obtained in 3 patients. Sympathoinhibition was observed during VF in 2 patients while sympathoexcitation was noted in the remaining patient. Figs. 11A-11C show a muscle SNA recording from one of the patients who developed sympathoinhibition during VF. Almost complete sympathetic shut down was noted when compared to baseline. SNCL did not change and even lengthened by 3% in the patient with sympathoinhibition whereas it shortened by 13% in the patient with sympathoexcitation. Our findings of sympathoinhibition associated with SNCL lengthening suggest the presence of vasovagal like physiology during VF in a subgroup of patients.

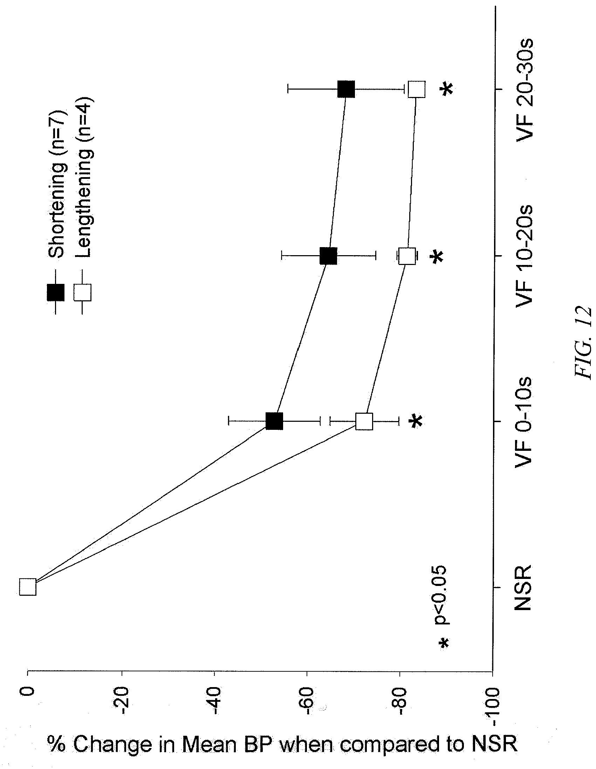

We also sought to determine the role of the autonomic changes during FVA in predicting blood pressure response and time to symptoms' onset. A test was conducted to determine that SNCL lengthening during FVA is associated with a greater decrease in blood pressure when compared to those with SNCL shortening. In 11 anesthetized pigs, the chest was opened and the heart was exposed on a pericardial cradle. A 247-electrode sock was placed around the ventricles, an atrial lead over the right atrial appendage and an arterial catheter in the right femoral artery for blood pressure monitoring. Using DC current or the shock-on-T method, VF was induced and sustained for 30 sec. before applying a DC shock. Animals were divided in to 2 groups based on SNCL response during VF: Shortening group (n=7, %ΔSNCL= -17%), Lengthening group (n=4, %ΔSNCL=4%). In the Shortening group, mean BP fell by 53% in the first 10 sec. when compared to NSR and continued to fall to 64% and 68% at 20 sec. and 30 sec. respectively. In the Lengthening group, BP fell by 72%, 81% and 83% when compared to baseline. The differences in percent decrease in BP between the groups were statistically significant at all 3 time points during VF (p<0.05). SNCL lengthening was associated with a greater decrease in BP when compared to SNCL shortening. The above findings suggest that SNCL changes during VF might be helpful in predicting the magnitude of the hemodynamic fall in BP. Such information could be useful in deciding who has "time" for painless therapy before delivering shock therapy in patients with dual chamber ICDs. SNCL lengthening was associated with a greater decrease in BP when compared to SNCL shortening. Figure 12 depicts changes in MBP when compared to NSR during 10-second increments of a 30-second period of AF.

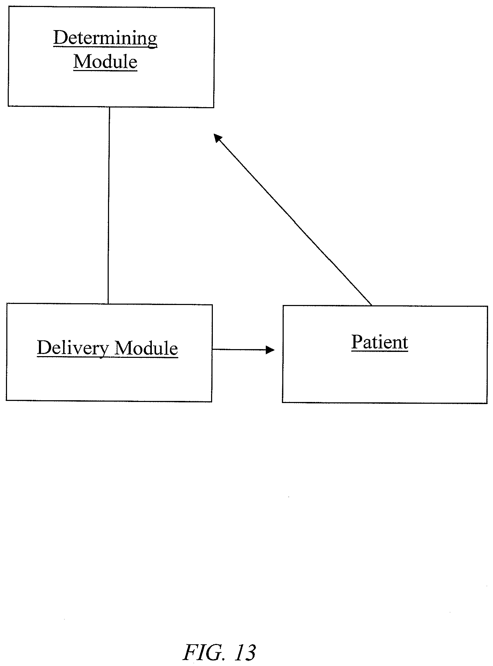

Fig. 13 depicts a determining module that can, for, example, determine a value of a parameter indicative of a rate of an intrinsic pacemaker of a heart of a patient experiencing a FVA. Also depicted is a delivery module, programmed to deliver a first therapy, for terminating the FVA, to the patient if the value indicates the depolarization rate is about equal to or higher than a threshold, and to deliver a second therapy, for terminating the FVA, different from the first therapy, to the patient if the value indicates the depolarization rate is lower than the threshold. In some embodiments, the determining module receives information from the patient, as indicated in Fig. 13. Fig. 13 also shows delivery of therapy from the delivery module to the patient.

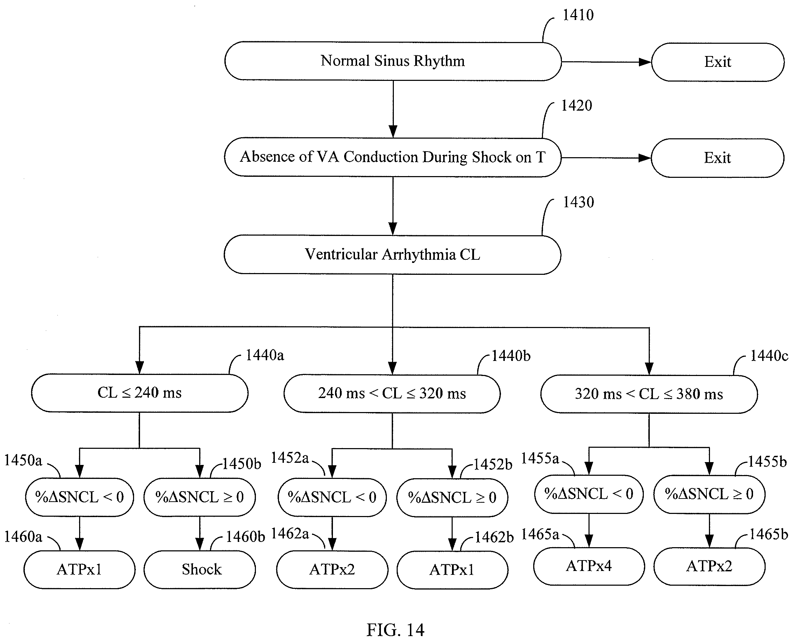

Fig. 14 is a flowchart of a method of operation of an implantable cardiac device for treating an abnormal heart rhythm, such as ventricular arrhythmia. The method may be used to determine when it is appropriate to treat the abnormal heart rhythm, such as ventricular arrhythmia, with painless therapy, such as ATP, instead of shock therapy. As discussed above, while shock therapy is effective at terminating ventricular arrhythmia, shock therapy involves the delivery of painful shocks to the patient, which negatively impacts the quality of life of the patient. In addition, shock therapy may lead to myocardial damage and quickly drain the battery of an ICD. Thus, it can be desirable to treat ventricular arrhythmia with painless therapy in cases where the painless therapy will be effective to avoid subjecting the patient to painful shocks. However, if the painless therapy fails, the patient may lose consciousness. In addition, the failed treatment attempt delays the use of shock therapy. The delay may decrease the success rate of subsequent shocks.

In one embodiment, the method determines a change in the sinus node cycle length (SNCL) of the patient's heart between a time prior to the onset of ventricular arrhythmia and a time during the ventricular arrhythmia. It is believed that SNCL lengthening during ventricular arrhythmia correlates with a more rapid drop in blood pressure and a shorter time to syncope compared to SNCL shortening. As a result, for a patient with SNCL lengthening during ventricular arrhythmia, there be may less time to attempt painless therapy before syncope and a lower likelihood of success of painless therapy. Thus, the method may use the change in SNCL to predict the likelihood of success of painless therapy and to determine whether to use painless therapy to treat a particular episode of ventricular arrhythmia, as discussed further below.

A method of operation of an implantable cardiac device for treating ventricular arrhythmia according to various embodiments will now be described with reference to Fig. 14. The method may include determining a change in the SNCL of a heart of a patient between a time prior to an abnormal heart rhythm and a time during the abnormal heart rhythm, and determining which one of a plurality of different therapies to deliver to the patient to treat the abnormal heart rhythm based on the change in the SNCL.

In step 1410, the method determines whether the heart has a normal sinus rhythm. This may be determined by sensing electrical activity of the heart using one or more implanted electrodes. If the heart has a normal sinus rhythm, then the method proceeds to step 1420. Otherwise, the method is exited.

In step 1420, the method determines whether Ventriculoatrial (VA) conduction is absent during the induction of VF using the shock on T method. VA conduction may occur when electrical signals propagate from the ventricle to the upper chambers of the heart. These electrical signals may result in electrical noise in the upper chambers of the heart that prevents an accurate determination of the sinus node cycle length. The shock on T may be used to artificially induce an abnormal heart rhythm when an ICD is first implanted in the patient to test whether the ICD is functional. If VA conduction is absent during the shock on T, then the method proceeds to step 1430. Otherwise the method is exited. After implantation of the IDC, step 1420 may be omitted.

In step 1430, the method determines whether the heart is experiencing a ventricular arrhythmia. This may be done by measuring a rate of electrical impulses in the ventricle using one or more implanted electrodes and detecting the ventricular arrhythmia when the rate exceeds a threshold. Other methods known in the art may also be used to detect ventricular arrhythmias. The method also determines the cycle length of the ventricular arrhythmia. This may be done by measuring the time between consecutive impulses in a ventricle or other method.

The method then proceeds to one of steps 1440a-1440c depending on the cycle length of the ventricular arrhythmia. When the cycle length is less than about 240 ms, corresponding to rapid ventricular arrhythmia, the method proceeds to step 1440a. When the cycle length is between about 320 ms and about 240 ms, the method proceeds to step 1440b. Finally, when the cycle length is between about 380 ms and about 320 ms, the method proceeds to step 1440c. The cycle length ranges in Fig. 14 are exemplary only and other cycle length ranges may be used.

In step 1440a, the method determines a change in the SNCL between a time prior to the onset of the ventricular arrhythmia and a time during the ventricular arrhythmia. In one embodiment, the change in the SNCL may be given as a percent SNCL change (%ΔSNCL). The percent SNCL change may be defined as %ΔSNCL = (VA-SNCL-Baseline-SNCL)/(Baseline-SNCL)*100, where VA-SNCL is the SNCL during the ventricular arrhythmia and Baseline-SNCL is the SNCL prior to the onset of the ventricular arrhythmia. The VA-SNCL may be obtained by computing the mean SNCL during a time window (e.g., 10 seconds or less) after the onset of the ventricular arrhythmia. The Baseline-SNCL may be obtained by computing the mean SNCL during a time window (e.g., 60 seconds or less) prior to the onset of the ventricular arrhythmia. The SNCL may be determined by measuring the time between consecutive electrical impulses in the upper chambers of the heart using one or more implanted electrodes.

When %ΔSNCL is less than zero, the method proceeds to step 1450a. This occurs when the SNCL shortens during ventricular arrhythmia compared to the time prior to the ventricular arrhythmia. In this case, the method delivers ATP to the patient to treat the ventricular arrhythmia in step 1460a. The ATP may comprise a sequence of pulses delivered to one or both ventricles of the heart using one or more implanted electrodes.

When %ΔSNCL is equal to or greater than zero, the method proceeds to step 1450b. This occurs when the SNCL does not change or lengthens during ventricular arrhythmia compared to the time prior to the ventricular arrhythmia. As discussed above, the absence of SNCL changes or the presence of SNCL lengthening correlates with a more rapid drop in blood pressure and shortened time to syncope compared to SNCL shortening. In this case, the method delivers shock therapy to the patient to treat the ventricular arrhythmia in step 1460b without attempting ATP first. The shock therapy may include one of defibrillation and electrical cardioversion.

The ranges shown in Fig. 14 for the change in SNCL are exemplary. For example, the method may proceed to step 1450a when the percent SNCL change %ΔSNCL is less than a negative value, e.g., less than -10%, and proceed to step 1450a when %ΔSNCL is above this value. In other words, the boundary between whether to deliver ATP or shock therapy to the patient may be a value other than zero. In general, the method may proceed to step 1450a when the change in SNCL is within a first range and proceed to step 1450b when the change in SNCL is within a second range, different from the first range. The same applies to steps 1452a, 142b, 1455a and 1455b discussed below.

In step 1440b, which corresponds to a ventricular arrhythmia cycle length of between 240 ms and 320 ms, the method determines a change in the SNCL between a time during the ventricular arrhythmia and a time prior to the onset of the ventricular arrhythmia. The change in the SNCL may be given as a percent SNCL change (%ΔSNCL), as discussed above.

When %ΔSNCL is less than zero, which corresponds to SNCL shortening, the method proceeds to step 1452a. In this case, the method may deliver ATP twice (ATPx2) to the patient to treat the ventricular arrhythmia in step 1462a. Each ATP may comprise a sequence of pulses (e.g., six, seven, eight, nine or ten pulses) with a CL ≤ 88 % of VA CL.

When %ΔSNCL is equal to or greater than zero, which corresponds to SNCL lengthening, the method proceeds to step 1452b. As discussed above, SNCL lengthening correlates with a shortened time to syncope compared to SNCL shortening, providing less time for ATP attempts before syncope. In the case, the method may deliver ATP once (ATPx1) to the patient to treat the ventricular arrhythmia in step 1462b. The ATP may comprise a sequence of pulses at 88 % of VA CL. If the ATP fails, then shock therapy may be delivered to the patient.

In step 1440c, which corresponds to a ventricular arrhythmia cycle length of between 320 ms and 380 ms, the method determines a change in the SNCL between a time during the ventricular arrhythmia and a time prior to the onset of ventricular arrhythmia. The change in the SNCL may be given as a percent SNCL change (%ΔSNCL).

When %ΔSNCL is less than zero, which corresponds to SNCL shortening, the method proceeds to step 1455a. In this case, the method may deliver ATP four times (ATPx4) to the patient to treat the ventricular arrhythmia in step 1465a. Each of the first two ATPs may comprise a sequence of pulses (e.g., eight pulses) at 88 % of VA CL and each of the last two ATPs may comprise a sequence of pulses (e.g., eight pulses) with a CL ≤ 88% of VA CL.

When %ΔSNCL is equal to or greater than zero, which corresponds to SNCL lengthening, the method proceeds to step 1455b. As discussed above, SNCL lengthening correlates with a shortened time to syncope compared to SNCL shortening, providing less time for ATP attempts before syncope. In this case, the method may deliver ATP twice (ATPx2) to the patient to treat the ventricular arrhythmia in step 1465b.

Thus, the method may examine both the ventricular arrhythmia cycle length and changes in the SNCL in determining the appropriate therapy to treat ventricular arrhythmia. First, the method may examine the ventricular arrhythmia cycle length and select a subset of possible therapies based on the cycle length. For example, when the cycle length is less than 240 ms, the method in Fig. 14 selects the subset of APTx1 (one ATP attempt) and shock therapy as possible therapies. When the cycle length is between 240 ms and 320, the method in Fig. 14 selects the subset ATPx2 (two ATP attempts) and APTx1. Those skilled in the art will appreciate that the cycle length ranges and possible therapies illustrated in Fig. 14 are exemplary. Second, the method selects one of the therapies from the subset of therapies based on the change in the SNCL between a time prior to the onset of the ventricular arrhythmia and a time during the ventricular arrhythmia. In one embodiment, the method selects the therapy to treat the ventricular arrhythmia based on whether the SNCL shortens (e.g., %ΔSNCL <0) or the SNCL lengthens (e.g., %ΔSNCL ≥ 0) during the ventricular arrhythmia compared to the time prior to the ventricular arrhythmia.

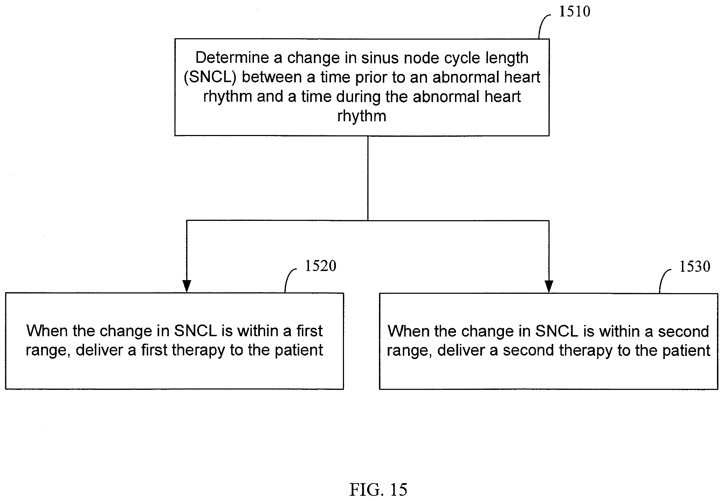

Fig. 15 is a flowchart of an exemplary method for treating an abnormal heart rhythm based on a change in SNCL. The abnormal heart rhythm may be ventricular arrhythmia. The abnormal heart rhythm may be detected by an ICD monitoring electrical activity of the heart. In step 1510, the method determines a change in the SNCL between a time prior to the abnormal rhythm and a time during the abnormal heart rhythm. When the change in SNCL is within a first range, the method delivers a first therapy to the patient in step 1520. For example, the first range may correspond to lengthening of the SNCL during the abnormal heart rhythm compared to the time prior to the abnormal heart rhythm. When the change in SNCL is within the second range, the method delivers a second therapy, different from the first therapy, to the patient in step 1530. For example, the second range may correspond to shortening of the SNCL during the abnormal heart rhythm compared to the time prior to the abnormal heart rhythm.

The concept of the present invention may be performed by an ICD implanted in the patient. For example, an ICD capable of delivering both ATP and shock therapy to a patient may be programmed to perform the methods according to various embodiments of the present invention. The ICD may comprise the determining module and delivery module shown in Fig. 13.

The determining module may comprise electrodes implanted in the patient for sensing electrical activity of the heart. The electrodes may include one or more electrodes positioned in or near the upper and/or lower chambers of the heart. The determining module may also include a processor for analyzing electrical measurements from the one or more electrodes and a machine-readable storage device for storing the measurements and one or more programs executed by the processor for performing the methods according to various embodiments of the invention. The machine-readable storage device may include RAM, ROM, flash memory or a combination thereof. In one embodiment, the determining module may continuously monitor the SNCL and store a moving window of the SNCL in memory (e.g., the SNCL in the last 5 seconds). When a ventricular arrhythmia or other abnormal heart rhythm is detected, the determining module can retrieve the stored SNCL to determine the SNCL prior to the onset of the ventricular arrhythmia. The determining module may then determine the change in the SNCL between the time prior to and during the ventricular arrhythmia. In one embodiment, the determining module may compute a first mean SNCL within a first window (e.g., 5 seconds) prior to the onset of the ventricular arrhythmia and compute a second mean SNCL within a second window (e.g., 5 seconds) after the onset of the ventricular arrhythmia. The determining module may then determine the change in SNCL based on the first and second mean SNCL.

The delivery module may include one or more electrodes implanted in the patient for delivering ATP or shock therapy to the patient. For example, the delivery module may include one or more electrodes implanted in or near one or both ventricles of the heart to deliver ATP or shock therapy to terminate ventricular arrhythmia. The delivery module may share one or more electrodes with the determining module. The delivery module may also include one or more electrical signal generators to generate electrical signals for the ATP or shock therapy. The delivery module may also include the same processor as the determining module or a different processor and the same memory as the determining module or a different memory. The delivery module may be programmed to receive the change in SNCL from the determining module and to deliver one of a plurality of different therapies to the patient based on the change in the SNCL. For example, the delivery module may be programmed to deliver a first therapy when the change in SNCL indicates SNCL lengthening during an abnormal heart rhythm compared to the time prior to the abnormal heart rhythm, and to deliver a second therapy, different from the first therapy, to the patient when the change in SNCL indicates SNCL shortening during the abnormal heart rhythm compared to the time prior to the abnormal heart rhythm.

Although preferred embodiments of the disclosure have been described in detail, certain variations and modifications will be apparent to those skilled in the art, and it is intended that the scope of the present disclosure herein disclosed should not be limited by the particular disclosed embodiments described above.

The scope of the invention is defined by the appended claims.

## Claims

1. An implantable cardiac device, for treating an abnormal heart rhythm, comprising:
a determining module configured to:
(i) determine occurrence of an abnormal heart rhythm comprising a ventricular arrhythmia, including determining a cycle length of the ventricular arrhythmia, and
(ii) determine a change in a sinus node cycle length of a heart of a patient from a time prior to onset of the abnormal heart rhythm to a time during the abnormal heart rhythm; and
a delivery module programmed to deliver to the patient, based on the determined change in the sinus node cycle length:
(i) a first electrical therapy comprising a first number of anti-tachycardia pacing attempts when the determined change in the sinus node cycle length corresponds to a lengthening of the sinus node cycle length during the abnormal heart rhythm, or
(ii) a second electrical therapy comprising a second number of anti-tachycardia pacing attempts when the determined change in the sinus node cycle length corresponds to a shortening of the sinus node cycle length during the abnormal heart rhythm,
wherein the second number of anti-tachycardia pacing attempts is greater than the first number of anti-tachycardia pacing attempts.

2. The device of Claim 1, wherein the determining module is configured to determine a first mean sinus node cycle length within a first time window prior to the onset of the abnormal heart rhythm, to determine a second mean sinus node cycle length within a second time window after the onset of the abnormal heart rhythm, and to determine the change in the sinus node cycle length based on the first and second mean sinus node cycle lengths.

3. The device of Claim 1, wherein the first electrical therapy further comprises at least one of defibrillation or electrical cardioversion without any anti-tachycardia pacing attempts.

4. The device of Claim 1, wherein each of the anti-tachycardia pacing attempts comprises a sequence of pulses delivered to a ventricle of the heart.

5. The device of Claim 1, wherein the first electrical therapy comprises at least one of defibrillation or electrical cardioversion, and wherein the first number of antitachycardia pacing attempts is zero, when both (a) the determined change indicates a lengthening of the sinus node cycle length and (b) the ventricular arrhythmia cycle length after the onset of the abnormal heart rhythm is less than or equal to about 240 ms.

6. The device of Claim 1, wherein the first electrical therapy comprises at least one antitachycardia pacing attempt when both (a) the determined change indicates a lengthening of the sinus node cycle length and (b) the ventricular arrhythmia cycle length after the onset of the abnormal heart rhythm is greater than about 240 ms.

7. The device of Claim 6, wherein, when both (a) the determined change indicates a lengthening of the sinus node cycle length and (b) the ventricular arrhythmia cycle length after the onset of the abnormal heart rhythm is greater than about 240 ms, the delivery module applies a greater number of antitachycardia pacing attempts when the ventricular arrhythmia cycle length after the onset of the abnormal heart rhythm is greater than 320 ms than when the ventricular arrhythmia cycle length after the onset of the abnormal heart rhythm is less than or equal to 320 ms.

8. The device of Claim 1, wherein, when the determined change indicates a shortening of the sinus node cycle length, the delivery module is configured to apply a number of antitachycardia pacing attempts selected based on a ventricular arrhythmia cycle length after the onset of the abnormal heart rhythm.

## Patentansprüche

1. Implantierbare Herzvorrichtung zum Behandeln eines abnormalen Herzrhythmus, die Folgendes umfasst:
ein Bestimmungsmodul, das dazu konfiguriert ist:
(i) das Auftreten eines eine ventrikuläre Arrhythmie umfassenden abnormalen Herzrhythmus zu bestimmen, einschließlich des Bestimmens einer Zykluslänge der ventrikulären Arrhythmie, und
(ii) eine Änderung einer Sinusknotenzykluslänge eines Herzens eines Patienten aus einer Zeit vor dem Einsetzen des abnormalen Herzrhythmus bis zu einer Zeit während des abnormalen Herzrhythmus zu bestimmen; und
ein Verabreichungsmodul, das dazu programmiert ist, dem Patienten basierend auf der bestimmten Änderung der Sinusknotenzykluslänge Folgendes zu verabreichen:
(i) eine erste Elektrotherapie, umfassend eine erste Zahl von antitachykarden Pacing-Versuchen, wenn die bestimmte Änderung der Sinusknotenzykluslänge einer Verlängerung der Sinusknotenzykluslänge während des abnormalen Herzrhythmus entspricht, oder
(ii) eine zweite Elektrotherapie, umfassend eine zweite Zahl von antitachykarden Pacing-Versuchen, wenn die bestimmte Änderung der Sinusknotenzykluslänge einer Verkürzung der Sinusknotenzykluslänge während des abnormalen Herzrhythmus entspricht,
wobei die zweite Zahl von antitachykarden Pacing-Versuchen größer ist als die erste Zahl von antitachykarden Pacing-Versuchen.

2. Vorrichtung nach Anspruch 1, wobei das Bestimmungsmodul dazu konfiguriert ist, eine erste mittlere Sinusknotenzykluslänge innerhalb eines ersten Zeitfensters vor dem Einsetzen des abnormalen Herzrhythmus zu bestimmen, eine zweite mittlere Sinusknotenzykluslänge innerhalb eines zweiten Zeitfensters nach dem Einsetzen des abnormalen Herzrhythmus zu bestimmen und die Änderung der Sinusknotenzykluslänge basierend auf der ersten und der zweiten Sinusknotenzykluslänge zu bestimmen.

3. Vorrichtung nach Anspruch 1, wobei die erste Elektrotherapie weiter Defibrillation und/oder Elektrokardioversion ohne antitachykarde Pacing-Versuche umfasst.

4. Vorrichtung nach Anspruch 1, wobei jeder der antitachykarden Pacing-Versuche eine Folge von an eine Herzkammer verabreichten Impulsen umfasst.

5. Vorrichtung nach Anspruch 1, wobei die erste Elektrotherapie Defibrillation und/oder Elektrokardioversion umfasst und wobei die erste Zahl von antitachykarden Pacing-Versuchen null ist, wenn (a) die bestimmte Änderung eine Verlängerung der Sinusknotenzykluslänge angibt und (b) die Zykluslänge der ventrikulären Arrhythmie nach dem Einsetzen des abnormalen Herzrhythmus geringer als oder gleich etwa 240 ms ist.

6. Vorrichtung nach Anspruch 1, wobei die erste Elektrotherapie mindestens einen antitachykarden Pacing-Versuch umfasst, wenn (a) die bestimmte Änderung eine Verlängerung der Sinusknotenzykluslänge angibt und (b) die Zykluslänge der ventrikulären Arrhythmie nach dem Einsetzen des abnormalen Herzrhythmus größer als etwa 240 ms ist.

7. Vorrichtung nach Anspruch 6, wobei, wenn (a) die bestimmte Änderung eine Verlängerung der Sinusknotenzykluslänge angibt und (b) die Zykluslänge der ventrikulären Arrhythmie nach dem Einsetzen des abnormalen Herzrhythmus größer als etwa 240 ms ist, das Verabreichungsmodul eine größere Zahl von antitachykarden Pacing-Versuchen anwendet, wenn die Zykluslänge der ventrikulären Arrhythmie nach dem Einsetzen des abnormalen Herzrhythmus größer als 320 ms ist, als wenn die Zykluslänge der ventrikulären Arrhythmie nach dem Einsetzen des abnormalen Herzrhythmus geringer als oder gleich 320 ms ist.

8. Vorrichtung nach Anspruch 1, wobei, wenn die bestimmte Änderung eine Verkürzung der Sinusknotenzykluslänge angibt, das Verabreichungsmodul dazu konfiguriert ist, eine Zahl von antitachykarden Pacing-Versuchen anzuwenden, die basierend auf einer Zykluslänge der ventrikulären Arrhythmie nach dem Einsetzen des abnormalen Herzrhythmus ausgewählt wird.

## Revendications

1. Dispositif cardiaque implantable, pour traiter un rythme cardiaque anormal, comprenant :
un module de détermination configuré pour :
(i) déterminer l'apparition d'un rythme cardiaque anormal comprenant une arythmie ventriculaire, incluant la détermination d'une longueur de cycle de l'arythmie ventriculaire, et
(ii) déterminer une variation d'une longueur de cycle de noeud sinusal d'un coeur d'un patient d'un temps avant le début du rythme cardiaque anormal à un temps pendant le rythme cardiaque anomal ; et
un module de distribution programmé pour distribuer au patient, sur la base de la variation déterminée de la longueur de cycle de noeud sinusal :
(i) une première thérapie électrique comprenant un premier nombre de tentatives de stimulation antitachycardique quand la variation déterminée de la longueur de cycle de noeud sinusal correspond à un allongement de la longueur de cycle de noeud sinusal pendant le rythme cardiaque anormal, ou
(ii) une deuxième thérapie électrique comprenant un deuxième nombre de tentatives de stimulation antitachycardique quand la variation déterminée de la longueur de cycle de noeud sinusal correspond à une réduction de la longueur de cycle de noeud sinusal pendant le rythme cardiaque anormal,
dans lequel le deuxième nombre de tentatives de stimulation antitachycardique est supérieur au premier nombre de tentatives de stimulation antitachycardique.

2. Dispositif selon la revendication 1, dans lequel le module de détermination est configuré pour déterminer une première longueur moyenne de cycle de noeud sinusal au sein d'une première fenêtre de temps avant le début du rythme cardiaque anormal, pour déterminer une deuxième longueur moyenne de cycle de noeud sinusal au sein d'une deuxième fenêtre de temps après le début du rythme cardiaque anormal, et pour déterminer la variation de la longueur de cycle de noeud sinusal sur la base des première et deuxième longueurs moyennes de cycle de noeud sinusal.

3. Dispositif selon la revendication 1, dans lequel la première thérapie électrique comprend en outre au moins un élément parmi la défibrillation ou la cardioversion électrique sans aucune tentative de stimulation antitachycardique.

4. Dispositif selon la revendication 1, dans lequel chacune des tentatives de stimulation antitachycardique comprend une séquence de pulsations distribuées à un ventricule du coeur.

5. Dispositif selon la revendication 1, dans lequel la première thérapie électrique comprend au moins un élément parmi la défibrillation ou la cardioversion électrique, et dans lequel le premier nombre de tentatives de stimulation antitachycardique est zéro, quand à la fois (a) la variation déterminée indique un allongement de la longueur de cycle de noeud sinusal et (b) la longueur de cycle d'arythmie ventriculaire après le début du rythme cardiaque anormal est inférieure ou égale à environ 240 ms.

6. Dispositif selon la revendication 1, dans lequel la première thérapie électrique comprend au moins une tentative de stimulation antitachycardique quand à la fois (a) la variation déterminée indique un allongement de la longueur de cycle de noeud sinusal et (b) la longueur de cycle d'arythmie ventriculaire après le début du rythme cardiaque anormal est supérieure à environ 240 ms.

7. Dispositif selon la revendication 6, dans lequel, quand à la fois (a) la variation déterminée indique un allongement de la longueur de cycle de noeud sinusal et (b) la longueur de cycle d'arythmie ventriculaire après le début du rythme cardiaque anormal est supérieure à environ 240 ms, le module de distribution applique un plus grand nombre de tentatives de stimulation antitachycardique quand la longueur de cycle d'arythmie ventriculaire après le début du rythme cardiaque anormal est supérieure à 320 ms que quand la longueur de cycle d'arythmie ventriculaire après le début du rythme cardiaque anormal est inférieure ou égale à 320 ms.

8. Dispositif selon la revendication 1, dans lequel, quand la variation déterminée indique une réduction de la longueur de cycle de noeud sinusal, le module de distribution est configuré pour appliquer un nombre de tentatives de stimulation antitachycardique sélectionné sur la base d'une longueur de cycle d'arythmie ventriculaire après le début du rythme cardiaque anormal.
